(19) Europäisches Patentamt
European Patent Office
Office européen des brevets

(11) Veröffentlichungsnummer: **0 028 270**
**B1**

(12) # EUROPÄISCHE PATENTSCHRIFT

(45) Veröffentlichungstag der Patentschrift: **16.02.83**

(21) Anmeldenummer: **79104314.4**

(22) Anmeldetag: **05.11.79**

(51) Int. Cl.³: **C 07 D 487/06,**
C 07 D 209/88,
A 61 K 31/495
//(C07D487/06, 209/00,
241/00)

(54) **Pyrazino-carbazole, Verfahren zu ihrer Herstellung und sie enthaltende pharmazeutische Mittel.**

(43) Veröffentlichungstag der Anmeldung:
**13.05.81 Patentblatt 81/19**

(45) Bekanntmachung des Hinweises auf die Patenterteilung:
**16.02.83 Patentblatt 83/7**

(84) Benannte Vertragsstaaten:
**AT CH DE FR GB IT NL**

(56) Entgegenhaltungen:
**DE - A - 2 250 493**
**US - A - 3 317 524**
**US - A - 3 542 780**

**Die Akte enthält technische Angaben, die nach dem Eingang der Anmeldung eingereicht wurden und die nicht in dieser Patentschrift enthalten sind.**

(73) Patentinhaber: **CASSELLA Aktiengesellschaft**
**Hanauer Landstrasse 526**
**D-6000 Frankfurt am Main 61 (DE)**

(72) Erfinder: **Bender, Heinz, Dr.**
**Kirchgasse 6**
**D-6000 Frankfurt am Main 60 (DE)**
Erfinder: **Beyerle, Rudi, Dr.**
**An der Pfaffenmauer 44**
**D-6000 Frankfurt am Main 60 (DE)**
Erfinder: **Keil, Karl-Heinz, Dr.**
**Konrad Adenauer Strasse 26**
**D-6050 Offenbach am Main (DE)**
Erfinder: **Greve, Heinz-Günter, Dr.**
**Birsteiner Strasse 45**
**D-6000 Frankfurt am Main 61 (DE)**
Erfinder: **Reh, Kuno, Dr.**
**Lauterbacher Strasse 14**
**D-6000 Frankfurt am Main 61 (DE)**
Erfinder: **Martorana, Piero Anton, Dr.**
**Kaiser-Friedrich-Promenade 108**
**D-6380 Bad Homburg vor der Höhe (DE)**
Erfinder: **Piesch, Steffen, Dr.**
**An der Heide 32**
**D-6370 Oberursel/Taunus (DE)**

(74) Vertreter: **Urbach, Hans-Georg, Dr. et al,**
**Hanauer Landstrasse 526**
**D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 028 270 B1

Pyrazino-carbazole, Verfahren zu ihrer Herstellung und sie enthaltende pharmazeutische Mittel

Die Erfindung betrifft pharmakologisch wertvolle, in 8-Stellung substituierte 2,4,5,6-Tetrahydro-1H-pyrazino-[3,2,1-jk]carbazole der Formel I

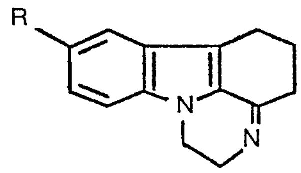

(I)

wobei R für eine Alkyl- oder Alkoxygruppe mit 1—4 Kohlenstoffatomen oder ein Fluor-, Chlor- oder Bromatom steht, sowie deren Säureadditionssalze, ihre Herstellung und die sie enthaltenden pharmazeutisch annehmbaren psychotropen Mittel.

Zur Bildung der pharmazeutisch annehmbaren Säureadditionssalze der Verbindungen der allgemeinen Formel I sind anorganische und organische Säuren geeignet, beispielsweise Chlorwasserstoff, Bromwasserstoff, Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diäthylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Citronen- oder Adipinsäure.

Die Säureadditionssalze können wie üblich durch Vereinigung der Komponenten, zweckmäßigerweise in einem geeigneten Verdünnungs- bzw. Dispergiermittel, erhalten werden.

Die Verbindungen der Formel I werden dadurch erhalten, daß man ein 6,9-disubstituiertes 1,2,3,4-tetrahydrocarbazol-1-on der Formel II

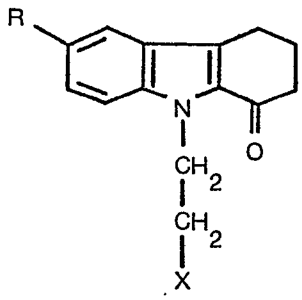

(II)

wobei R für eine Alkyl- oder Alkoxygruppe mit 1—4 Kohlenstoffatomen oder ein Fluor-, Chlor- oder Bromatom steht und X ein Brom-, Chlor- oder Jodatom oder den Rest der Formel $—O—SO_2—R'$ und R' einen Alkylrest mit 1—3 Kohlenstoffatomen, Phenyl oder Tolyl bedeutet, mit Ammoniak unter Ringschluß umsetzt.

Die als Ausgangsprodukte u.a. eingesetzten neuen 2,6-disubstituierten 1,2,3,4-Tetrahydrocarbazol-1-one der Formel IIa werden erhalten durch Umsetzung von in 6-Stellung substituierten 1,2,3,4-Tetrahydrocarbazol-1-onen der Formel III mit 1,2-Dihalogenäthanen oder den Sulfonsäureestern von 1-Hydroxy-2-halogenäthanen.

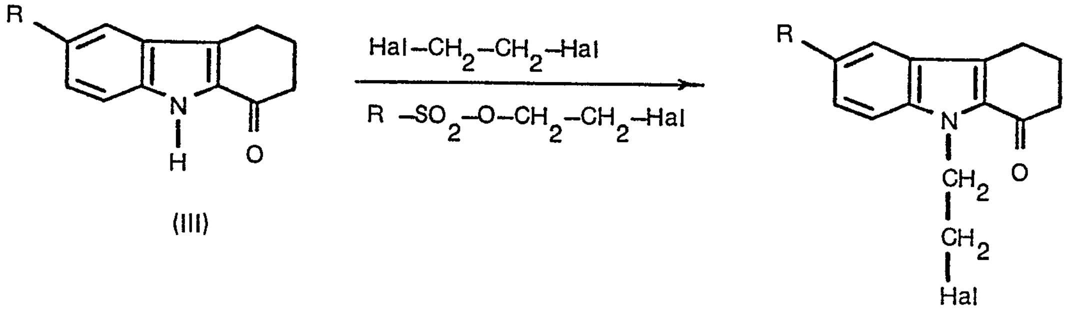


(III) (IIa)

Hier steht Hal für Chlor, Brom oder Jod und $R_1$ für Alkyl, Aryl oder Aralkyl. Als 1,2-Dihalogenäthane und als Sulfonsäureester von 1-Hydroxy-2-halogenäthanen sind demnach solche geeignet, die als Halogen Chlor, Brom oder Jod enthalten, beispielsweise 1,2-Dichlor-, 1,2-Dibrom- oder 1,2-Dijodäthan. Auch 1,2-Dihalogenäthane, die zwei verschiedene Halogenatome enthalten, wie z.B. 1-Brom-2-chlor-äthan, sind geeignet. In den Sulfonsäureestern der 1-Hydroxy-2-brom-, -chlor- oder

-jodäthane kann sich die Sulfonsäureestergruppe von einer Alkylsulfonsäure, insbesondere einer mit 1 bis 4 C-Atomen, beispielsweise Methan-, Äthan- oder n-Butansulfonsäure, einer Arylsulfonsäure, insbesondere mit 6 bis 12 C-Atomen, beispielsweise Benzol-, o-, m- oder o-Tolyl- oder 2,3-, 2,4- oder 3,5-Xylol- oder $\alpha$- oder $\beta$-Naphthylsulfonsäure, einer Aralkylsulfonsäure, insbesondere mit 7 oder 8 C-Atomen, wie z.B. Benzyl- oder Phenäthylsulfonsäure, ableiten.

Die Umsetzung der Tetrahydrocarbazolone der Formel III mit den 1,2-Dihalogenäthanen oder den Sulfonsäureestern der 1-Hydroxy-2-halogenäthane erfolgt am günstigsten unter den Bedingungen der Phasentransferkatalyse. Die Umsetzung erfolgt unter Rühren in einem 2-Phasensystem aus Wasser und einem mit Wasser nicht mischbaren organischen Lösungsmittel in Gegenwart einer starken Base und eines Phasentransferkatalysators.

Als organische Lösungsmittel sind mit Wasser nicht mischbare Lösungsmittel geeignet, die sich unter den Reaktionsbedingungen inert verhalten, d.h. nicht störend in die Reaktion eingreifen. Geeignete Lösungsmittel sind z.B. aliphatische, cycloaliphatische oder aromatische Kohlenwasserstoffe und aromatische Halogenkohlenwasserstoffe, wie z.B. Petroläther, Benzol, Toluol, 1,2-, 1,3- oder 1,4-Xylol, Chlorbenzol. Auch Mischungen der genannten Lösungsmittel können verwendet werden. Anstelle des mit Wasser nicht mischbaren organischen Lösungsmittels kann auch überschüssiges 1,2-Dihalogenäthan eingesetzt werden. Als starke Base ist z.B. Natrium- oder Kaliumhydroxid geeignet. Es können die üblichen Phasentransferkatalysatoren verwendet werden. Die bekannten Phasentransferkatalysatoren sind quartäre Ammonium- oder Phosphoniumsalze, wobei im Kation zweckmäßigerweise mindestens ein hydrophober Rest mit 4 oder mehr C-Atomen vorhanden ist. Ein derartiger Rest kann ein Alkyl-, Aryl- oder Aralkylrest sein. Geeignete Verbindungen sind z.B. Tetrabutylammoniumhydrogensulfat, Benzyltrimethylammoniumchlorid und ähnliche Verbindungen. Kationen weiterer bekannter Phasentransferkatalysatoren sind z.B. Tetrapropylammonium, Tetradodecylammonium, Benzyltriäthylammonium, Trihexylmethylammonium, Cetyltrimethylammonium, n-Alkyltriäthylammonium, wobei der Alkylrest 4 oder mehr, beispielsweise 6 C-Atome besitzt, Trioctylmethylammonium, Tricaprylylmethylammonium (Kation des Handelsprodukts Aliquat 336 der Fa. Gendval Mills. Comp., Kankakee, Ill/USA), Hexadecyltributylphosphonium. Geeignete Anionen für diese Kationen sind insbesondere Hydrogensulfat, Chlorid und Bromid.

Bei der Durchführung der Reaktion ist eine Erwärmung der Reaktionsmischung normalerweise nicht erforderlich. Zu Beginn erfolgt meist eine leichte Temperaturerhöhung infolge der einsetzenden Reaktion. Die Reaktionszeiten betragen bei Umgebungstemperatur etwa 1 bis 40 Stunden, häufig bis 20 Stunden; sie können durch Erhöhung der Reaktionstemperatur, z.B. auf 60°C, verringert werden.

Bezogen auf 1 Mol Tetrahydrocarbazolon der Formel III werden als Reaktionskomponente normalerweise 1 bis 3 Mol, vorzugsweise 1 bis 2 Mol 1,2-Dihalogenäthan oder Sulfonsäureester des 1-Hydroxy-2-halogenäthans eingesetzt. Falls 1,2-Dihalogenäthan nicht nur als Reaktionskomponente, sondern auch als Lösungsmittel dient, ist selbstverständlich ein höherer Überschuß erforderlich. In diesem Fall können pro Mol Tetrahydrocarbazolon der Formel III beispielsweise bis zu 15 Mol und gegebenenfalls noch mehr, vorzugsweise bis zu 10 Mol, 1,2-Dihalogenäthan eingesetzt werden. Pro Mol Tetrahydrocarbazolon der Formel III werden normalerweise 4 bis 15 Mole, vorzugsweise 5 bis 10 Mole, der starken Base, zweckmäßigerweise in Form einer wäßrigen Lösung, eingesetzt, deren Konzentration in der Regel 10 bis 40 Gew.%, vorzugsweise 20 bis 40 Gew.%, beträgt. Von dem Katalysator werden pro Mol Tetrahydrocarbazolon der Formel III in der Regel 0,005 bis 0,05 Mole, vorzugsweise 0,005 bis 0,02 Mole, benötigt. Der Katalysator wird zweckmäßig in etwas Wasser gelöst zugesetzt. Selbstverständlich könnten von dem 1,2-Dihalogenäthan bzw. dem Sulfonsäureester des 1-Hydroxy-2-halogenäthans der starken Base und dem Katalysator auch größere als die vorgenannten Mengen eingesetzt werden. Eine solche Erhöhung bringt aber keine Vorteile. Eine Verringerung der Menge der starken Base und des Katalysators ist häufig ebenfalls möglich. Dabei ist jedoch zu beachten, daß bei einer Verringerung der Katalysatormenge die Reaktionszeit zunimmt. Bei Katalysatormengen kleiner als 5 mMol, bezogen auf 1 Mol der Verbindung der Formel III, werden die Reaktionszeiten zumeist unwirtschaftlich lang.

Die als Ausgangsprodukte ferner eingesetzten neuen Alkyl- bzw. Arylsulfonsäureester der Formel IIb werden erhalten, indem man 1,2,3,4-Tetrahydrocarbazol-1-one der Formel III in sehr guter Ausbeute mit Äthylenoxid zu den 9-(Hydroxyäthyl)-1,2,3,4-tetrahydrocarbazol-1-onen der Formel IV umsetzt und anschliessend in an sich bekannter Weise in die Alkyl- oder Arylsulfonsäureester der Formel IIb überführt.

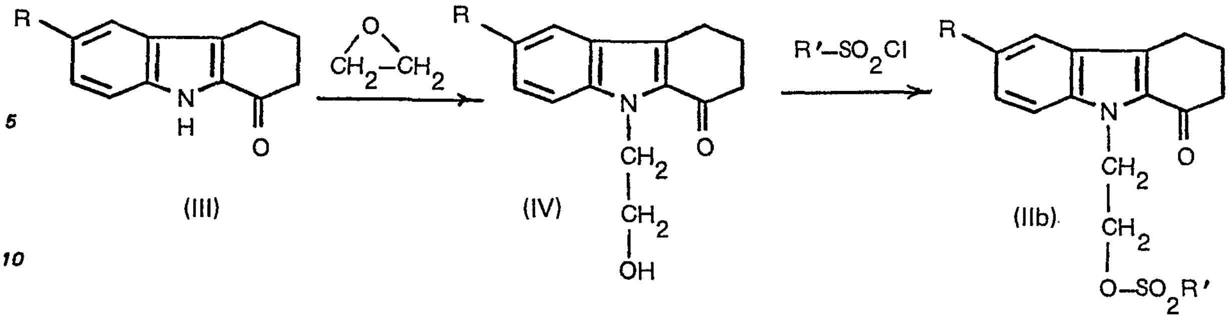


(III) (IV) (IIb)

Die Aethoxylierung der 1,2,3,4-Tetrahydrocarbazol-1-one (Formel III) zu den 9-Hydroxyäthyl-1,2,3,4-tetrahydrocarbazol-1-onen (Formel IV) wird in Gegenwart von alkalischen Katalysatoren, wie Natriumhydroxid, Kaliumhydroxid etc. in Lösungsmitteln, die gegenüber den Reaktionspartnern inert sind, durchgeführt. Als besonders geeignet erwiesen sich dipolare aprotische Lösungsmittel wie Dimethylformamid und Dimethylsulfoxyd. Als vorteilhaft hat sich die Anwesenheit von Wasser erwiesen, durch das die Entstehung von Nebenprodukten vermindert wird.

Zur Überführung der 9-Hydroxyäthyl-1,2,3,4-tetrahydrocarbazol-1-one der Formel IV in die Sulfonsäureester der Formel IIb sind praktisch alle aromatischen oder aliphatischen Sulfonsäurechloride geeignet. Bevorzugt wird Methansulfochlorid verwendet. Man arbeitet in indifferenten Lösungsmitteln, insbesondere in Kohlenwasserstoffen, wie Toluol oder Benzol, und in Gegenwart von tertiären Basen, wie Triäthylamin oder Pyridin.

Die als Ausgangsverbindungen benötigten, in 6-Stellung durch Alkyl, Alkoxy oder Halogen substituierten 1,2,3,4-Tetrahydrocarbazol-1-one der Formel III sind, soweit sie nicht bereits bekannt sind, leicht nach bekannten Verfahren für die Herstellung von 1,2,3,4-Tetrahydrocarbazolonen zu synthetisieren. Bei den bekannten Verfahren, z.B. von Sen und Gosh, Journ. ind. chem. Soc. *4* (1927) 477 bis 491, und von Coffey, Rec. trav. chim. Pay-Bas, *42* (1923) 528 bis 532, wird aus Cyclohexanon das 2-Hydroxymethylen-cyclohexanon synthetisiert und dieses mit Diazobenzolchlorid in das Cyclohexan-1,2-dion-monophenylhydrazon überführt, das unter Einwirkung von heißer Essigsäure in das 1,2,3,4-Tetrahydrocarbazol-1-on übergeht. Zur Herstellung der benötigten Ausgangsverbindungen der Formel III brauchen bei dem bekannten Verfahren lediglich Diazobenzolchloride eingesetzt werden, die im Benzolkern in der gewünschten Weise substituiert sind. Die gewünschten Ausgangsprodukte der Formel III können auch nach dem Verfahren von Bloink und Pausacker Soc., 1950, Seiten 1328 bis 1331, aus 2-Hydroxycyclohexanon mit entsprechend im Phenylkern substituiertem Phenylhydrazin hergestellt werden.

Der Ringschluß der in 6-Stellung durch Alkyl, Alkoxy oder Halogen substituierten 9-Halogenäthyl-1,2,3,4-tetrahydrocarbazol 1-one der Formel IIa bzw. der 9-Alkyl- bzw. Aryl-sulfonyloxyäthyl-1,2,3,4-tetrahydro-carbazol-1-one der Formel IIb zu den erfindungsgemäßen Verbindungen der Formel I wird mit Ammoniak vorgenommen. Hierzu sind pro Mol der Verbindung II theoretisch mindestens 2 Mole Ammoniak erforderlich. Die Durchführung der Reaktion wird jedoch erleichtert, wenn ein Überschuß von Ammoniak verwendet wird, der z.B. das 10- bis 70-fache, vorzugsweise das 20- bis 50-fache, der theoretisch erforderlichen Menge beträgt. Der Ammoniak kann in Form einer Lösung in Wasser oder in einem organischen Lösungsmittel, das sich unter den Reaktionsbedingungen inert verhält, eingesetzt werden. Geeignete organische Lösungsmittel für den Ammoniak sind z.B. Alkohole, wie z.B. Methanol und Äthanol. Vorzugsweise wird jedoch flüssiger Ammoniak verwendet. Bei der Ringschlußraktion wird der Reaktionsansatz zweckmäßigerweise gerührt. Insbesondere bei der bevorzugten Verwendung von flüssigem Ammoniak ist es zur Beschleunigung der Reaktion zweckmäßig, die Reaktionstemperatur z.B. auf 50 bis 100°C zu erhöhen. Bei Verwendung von flüssigem Ammoniak wird die Reaktion dann in einem Autoklaven durchgeführt.

Die erfindungsgemäßen Verbindungen der allgemeinen Formel I und deren pharmazeutisch annehmbaren Säureadditionssalze besitzen wertvolle pharmazeutische Eigenschaften. Sie besitzen vor allem eine stark ausgeprägte psychotrope, insbesondere antidepressive Wirkung.

Die erfindungsgemäßen Verbindungen der Formel I und ihre pharmazeutisch annehmbaren Säureadditionssalze können daher am Menschen für sich allein, in Mischungen untereinander oder in pharmazeutischen Zubereitungen als psychotrope Mittel, insbesondere als Antidepressiva, verabreicht werden, die als aktiven Bestandteil eine wirksame Dosis mindestens einer erfindungsgemäßen Verbindung der Formel I oder eines Säureadditionssalzes davon neben üblichen pharmazeutisch einwandfreien Träger- und Zusatzstoffen enthalten. Geeignete Trägerstoffe sind z.B. Wasser, pflanzliche Öle, Stärke, Gelatine, Milchzucker, Magnesiumstearat, Wachse, Vaseline etc. Als Zusatzstoffe können z.B. Netzmittel, Sprengmittel, Konservierungsmittel etc. verwendet werden. Die pharmazeutischen Präparate können in Form von z.B. Tabletten, Kapseln, wäßrigen oder öligen Lösungen oder Suspensionen, Emulsionen, injizierbaren wäßrigen oder öligen Lösungen oder Suspensionen, dispergierbaren Pulvern oder Aerosolmischungen vorliegen. Die pharmazeutischen Präparate können neben den Verbindungen der Formel I auch noch eine oder mehrere andere pharmazeutisch wirksame Substanzen, bei-

spielsweise Tranquilizer, wie z.B. Meprobamat, Chlorpromazine und Benzodiazepinsedativa, wie z.B. Diazepam oder Chlordiazepoxid; cardiovasculäre Mittel und Vasodilatoren, wie z.B. $\beta$-Blocker, Glyzerintrinitrat, Pentaerythrittetranitrat, Molsidomin und Carbochromen enthalten.

Die erfindungsgemäßen Verbindungen der Formel I sind als Antidepressiva bekannten Verbindungen dieser Art deutlich überlegen. Außerdem sind sie wertvolle Zwischenprodukte für die Herstellung anderer, ebenfalls pharmakologisch aktiver Verbindungen.

Die pharmakologische Überlegenheit der erfindungsgemäßen Verbindungen gegenüber einer bekannten struktur- und wirkungsähnlichen Verbindung ist aus der nachfolgenden Tabelle zu ersehen.

| Präparat | $LD_{50}$ | Tetrabenazin Ptosis Antagonismus $ED_{50}$ | $\frac{LD_{50}}{ED_{50}}$ |
|---|---|---|---|
| 8-Methyl-2,4,5,6-tetrahydro-1H-pyrazino[3,2,1-jk]carbazol-Hydrochlorid Beispiel 1 | 72 | 4 | 18,0 |
| 8-Methoxy-2,4,5,6-tetrahydro-1H-pyrazino[3,2,1-jk]carbazol-Hydrochlorid Beispiel 2 | 37 | 2 | 18,5 |
| 8-Methyl-2,3,3a,4,5,6-hexahydro-1H-pyrazino[3,2,1-jk]-carbazol-Hydrochlorid gemäß DOS 21 14 230 | 122 | 14 | 8,7 |

Die Herstellung der erfindungsgemäßen Verbindungen wird an folgenden Beispielen näher erläutert:

In den folgenden Beispielen bedeuten, sofern nichts anderes angegeben, Teile Gewichtsteile, Prozentangaben Gewichtsprozente, und die Temperaturen sind in Celsiusgraden angegeben.

## Beispiel 1

306 g (1 Mol) 6-Methyl-9-(2-bromäthyl)-1,2,3,4-tetrahydrocarbazol-1-on, 1500 ml (60 Mol) flüssiges Ammoniak werden in einem V2A-Autoklaven 20 Stunden auf 80°C erhitzt. Nach dem Verdampfen des überschüssigen Ammoniaks wird das zurückbleibende Rohprodukt in einem Gemisch von 2000 ml Essigsäureäthylester und 1000 ml Wasser suspendiert und so viel Natronlauge zugesetzt, bis die wäßrige Schicht deutlich alkalisch reagiert. Man schüttelt kräftig durch, wäscht die organische Schicht mit Wasser, trocknet mit Natriumsulfat und fällt das Hydrochlorid des 8-Methyl-2,4,5,6-tetrahydro-1H-pyrazino[3,2,1-jk]carbazol durch Einleiten von trockenem HCl-Gas aus.

Man saugt ab und kristallisiert aus einem Gemisch Essigester-Äthanol (1 : 1) um. Fp: 274 bis 276°C; Ausbeute 87% d.Th.; Molgewicht: 260,5.

$C_{15}H_{16}N_2 \cdot HCl$

| | C | H | N | $Cl^{\ominus}$ |
|---|---|---|---|---|
| berechnet: | 69,2 | 6,5 | 10,7 | 13,6 |
| gefunden: | 68,7 | 6,9 | 10,4 | 13,1 |

Anstelle des flüssigen Ammoniaks kann man auch Lösungen von Ammoniak in organischen Lösungsmitteln, die unter den Reaktionsbedingungen gegen Ammoniak indifferent sind oder Lösungen von Ammoniak in Wasser verwenden.

Herstellung des Ausgangsproduktes

6-Methyl-9-(2-bromäthyl)-1,2,3,4-tetrahydrocarbazol-1-on:

199 g (1 Mol) 6-Methyl-1,2,3,4-tetrahydrocarbazol-1-on (Fp: 199°C, Literaturangaben: 194—195°C, 195—196°C, 195°C, vgl, Bloink and Pausacker loc. cit, Seite 1330), 1520 g (8,1 Mol) 1,2-Dibromäthan und 1000 ml (7,5 Mol) wäßrige Natronlauge (30%) werden vorgelegt und unter Rühren bei Raumtemperatur 4,25 g (12,5 mMol) Tetrabutylammoniumhydrogensulfat, gelöst in 25 ml Wasser, zugesetzt. Unter leichtem Temperaturanstieg setzt die Reaktion ein, die unter ständigem Rühren nach etwa 7 Stunden zu Ende ist. Zur Aufarbeitung wird die wäßrigalkalische Schicht abgetrennt, die organische Schicht mit Wasser gewaschen und das überschüssige Dibromäthan im Vakuum abdestilliert. Der Rückstand wird aus 200 ml Äthanol umkristallisiert. Man erhält 281 g = 92% d.Th. 6-Methyl-9-(2-bromäthyl)-1,2,3,4-tetrahydrocarbazol-1-on mit einem Fp von 103°C. Die Menge des als Katalysator dienenden Tetrabutylammoniumhydrogensulfats kann bis auf 5 mMol pro 1 Mol eingesetztes Carbazolon verringert werden. Die Reaktionszeit verlängert sich hierbei auf das Doppelte.

Anstelle des überschüssigen Dibromäthans kann man auch andere, mit Wasser nicht mischbare und unter den Bedingungen der Reaktion indifferente Lösungsmittel, z.B. Toluol, Benzol oder Chlorbenzol verwenden:

199 g (1 Mol) 6-Methyl-1,2,3,4-tetrahydro-carbazol-1-on und 376 g (2 Mol) 1,2-Dibromäthan werden in 1000 ml Toluol gelöst und mit 1000 ml Natronlauge und 4,25 g (12,5 mMol) Tetrabutylammoniumhydrogensulfat 10 Stunden bei 60°C gerührt. Nach dem Abtrennen der wäßrigen Schicht, Abdestillieren des Lösungsmittels und Umkristallisieren des hierbei zurückbleibenden Rohprodukts erhält man 275 g 6-Methyl-9-(2-bromäthyl)-1,2,3,4-tetrahydrocarbazol-1-on (90% d.Th.) Fp.: 102 bis 103°C.

$C_{15}H_{16}BrNO$: Molgewicht 306

| | C | H | Br | N | O |
|---|---|---|---|---|---|
| berechnet: | 58,8 | 5,2 | 26,1 | 4,6 | 5,2 |
| gefunden: | 59,0 | 5,4 | 25,5 | 4,6 | 5,6 |

Anstelle des 6-Methyl-9-(2-bromäthyl)-1,2,3,4-tetrahydrocarbazol-1-ons kann auch das entsprechende Chloräthylderivat eingesetzt werden, daß man wie folgt erhält:

100 g (0,5 Mol) 6-Methyl-1,2,3,4-tetrahydro-carbazol-1-on, 500 ml (4 Mol) Äthylenchlorid werden mit 500 ml wäßrige NaOH (30%) und 4,25 g (12,5 mMol) Tetrabutylammoniumhydrogensulfat bei 20 bis 30°C 20 Stunden lang gerührt. Es wird wie in Beispiel 1 beschrieben aufgearbeitet. Man erhält 112 g (= 86% d.Th.) 6-Methyl-9-(2-chloräthyl)-1,2,3,4-tetrahydro-carbazol-1-on mit einem Fp. von 108°C.

$C_{15}H_{16}ClNO$: Molgewicht 261,5

| | C | H | Cl | N | O |
|---|---|---|---|---|---|
| berechnet: | 68,9 | 6,1 | 13,6 | 5,4 | 6,1 |
| gefunden: | 69,4 | 6,1 | 13,1 | 5,5 | 6,3 |

Anstelle der 1,2-Dihalogenäthane kann man weiterhin auch die Sulfonsäureester der 1-Hydroxy-2-halogenäthane als Ausgangsprodukte verwenden, die man wie folgt erhält:

100 g (0,5 Mol) 6-Methyl-1,2,3,4-tetrahydro-carbazol-1-on und 234,5 g (1 Mol) p-Toluolsulfonsäure-$\beta$-chloräthylester werden in 700 ml Toluol gelöst und mit einer Lösung von 4,25 g (12,5 mMol) Tetrabutylammoniumhydrogensulfat in 500 ml wäßriger Natronlauge (30%) 10 Stunden lang bei Raumtemperatur gerührt. Die organische Schicht wird abgetrennt, mit Wasser gewaschen und eingedampft. Man erhält 108 g (= 83% d.Th.) 6-Methyl-9-(2-chloräthyl)-1,2,3,4-tetrahydrocarbazol-1-on.

Fp.: 106 bis 107°C.

## Beispiel 2

Man setzt 6-Methoxy-9-(2-bromäthyl)-1,2,3,4-tetrahydrocarbazol-1-on nach den Angaben des Beispiels 1 um und erhält 8-Methoxy -2,4,5,6-tetrahydro-1H-pyrazino[3,2,1-jk]carbazol-hydrochlorid. Fp.: 288°C; Ausbeute 86% d.Th.; Molgewicht: 276,5

$C_{15}H_{16}N_2O.HCl$

| | C | H | O | N | $Cl^{\ominus}$ |
|---|---|---|---|---|---|
| berechnet: | 65,2 | 6,1 | 5,8 | 10,0 | 12,9 |
| gefunden: | 65,3 | 6,1 | 5,7 | 10,2 | 12,8 |

Herstellung der Ausgangsprodukte:

a) 6-Methoxy-1,2,3,4-tetrahydrocarbazol-1-on

123 g (1 Mol) p-Methoxyanilin werden in 600 ml Wasser unter Zusatz von 260 g konzentrierter

Salzsäure gelöst. Nach dem Abkühlen auf 0°C werden 69 g Natriumnitrit, gelöst in 130 ml Wasser, zugetropft. Nach beendeter Diazotierung gibt man 350 ml Methanol und 148 g (1 Mol) des Natriumsalzes des 1-Hydroxymethylen-cyclohexanons hinzu und hält die Temperatur durch Eiszusatz unter +3°C. Durch Zusatz von 35,5 g wasserfreier Soda und 250 g kristallisiertem Natriumacetat stellt man einen pH-Wert von 5 bis 6 ein. Nach beendeter Kupplung (ca. 10 bis 12 Stunden) wird durch Zugabe von ca. 250 g konzentrierter Natronlauge auf pH 9 bis 9,5 gestellt, 3 Stunden bei Raumtemperatur nachgerührt, abgesaugt und mit Wasser alkalifrei gewaschen. Ausbeute 202 g (= 87% d.Th.), Fp.: 177°C, an Cyclohexandion-mono-p-methoxyphenylhydrazon.

| | C | H | N | O |
|---|---|---|---|---|
| berechnet: | 67,2 | 6,9 | 12,1 | 13,8 |
| gefunden: | 67,1 | 6,7 | 11,9 | 13,9 |

232 g (1 Mol) Cyclohexandion-mono-p-methoxyphenylhydrazon, 1200 ml Wasser, 350 g Schwefelsäure (65 Bé), 100 ml Äthanol werden 3 Stunden lang am Rückfluß gekocht. Nach dem Abkühlen wird abgesaugt und mit Wasser säurefrei gewaschen.
Fp.: 222°C; Ausbeute: 193 g (= 90% d.Th.) 6-Methoxy-1,2,3,4-tetrahydrocarbazol-1-on.

| | C | H | N | O |
|---|---|---|---|---|
| berechnet: | 72,6 | 6,0 | 14,9 | 6,5 |
| gefunden: | 72,1 | 5,9 | 14,5 | 6,6 |

Nach der vorstehenden Vorschrift lassen sich durch Einsatz anders substituierter Aniline andere, in 6-Stellung substituierte 1,2,3,4-Tetrahydro-carbazol-1-one herstellen. Für das 6-Methoxy-1,2,3,4-tetrahydro-carbazol-1-on ist von Bloink und Pausacker loc. cit., Seite 1330, ein Fp. von 180 bis 182°C angegeben, wobei jedoch die von den genannten Autoren beschriebene Verbindung noch 5 Mol Kristallbenzol enthält.

b) 6-Methoxy-9-(2-bromäthyl)-1,2,3,4-tetrahydrocarbazol-1-on

215 g (1 Mol) 6-Methoxy-1,2,3,4-tetrahydro-carbazol-1-on (Fp.: 222°C), 1500 g 1,2-Dibromäthan, 1000 ml wäßrige Natronlauge (30%) und 2 g Tetrabutylammoniumhydrogensulfat werden bei Raumtemperatur 20 Stunden gerührt und, wie in Beispiel 1 beschrieben, aufgearbeitet. Man erhält 286 g (= 89% d.Th.) 6-Methoxy-9-(2-bromäthyl)-1,2,3,4-tetrahydrocarbazol-1-on. Fp.: 119°C.

$C_{15}H_{16}BrNO_2$: Molgewicht 322

| | C | H | Br | N | O |
|---|---|---|---|---|---|
| berechnet: | 55,9 | 5,0 | 24,8 | 4,3 | 9,9 |
| gefunden: | 56,1 | 5,0 | 24,5 | 4,5 | 9,5 |

## Beispiel 3

Man setzt 6-Chloro-9-(2-bromäthyl)-1,2,3,4-tetrahydrocarbazol-1-on nach den Angaben des Beispiels 1 um und erhält 8-Chlor-2,4,5,6-tetrahydro-1H-pyrazino[3,2,1-jk]carbazol-hydrochlorid.

Fp.: 295 bis 305°C; Ausbeute 90% d.Th.; Molgewicht: 231

$C_{14}H_{13}ClN_2 \cdot HCl$

| | C | H | N | Cl | $Cl^{\ominus}$ |
|---|---|---|---|---|---|
| berechnet: | 59,8 | 5,0 | 10,0 | 25,2 | 12,6 |
| gefunden: | 59,8 | 5,1 | 9,6 | 24,6 | 11,9 |

Das Ausgangsprodukt 6-Chloro-9-(2-bromäthyl)-1,2,3,4-tetrahydrocarbazol-1-on erhält man durch Umsetzung von 6-Chlor-1,2,3,4-tetrahydrocarbazol-1-on (Fp.: 226°C) nach den Angaben des Beispiels 1 für die Herstellung der Ausgangsprodukte. 6-Chlor-9-(2-bromäthyl)-1,2,3,4-tetrahydro-

carbazol-1-on besitzt einen Fp.: 121°C und wird erhalten in einer Ausbeute von 86% d.Th.; Molgewicht: 326,5.

$C_{14}H_{13}BrClNO$

| | C | H | Br | Cl | N | O |
|---|---|---|---|---|---|---|
| berechnet: | 51,5 | 4,0 | 24,4 | 10,9 | 4,3 | 4,9 |
| gefunden: | 51,9 | 4,0 | 23,9 | 11,2 | 4,3 | 5,3 |

Beispiel 4

Man setzt 6-Brom-9-(2-bromäthyl)-1,2,3,4-tetrahydrocarbazol-1-on nach den Angaben des Beispiels 1 um und erhält 8-Brom-2,4,5,6-tetrahydro-1H-pyrazino[3,2,1-jk]carbazolhydrochlorid. Fp.: 298 bis 300°C; Ausbeute: 85% d.Th.; Molgewicht: 325,5.

$C_{14}H_{13}BrN_2.HCl$

| | C | H | N | Br | $Cl^{\ominus}$ |
|---|---|---|---|---|---|
| berechnet: | 51,8 | 4,3 | 8,6 | 24,6 | 10,9 |
| gefunden: | 51,7 | 4,3 | 8,4 | 24,2 | 10,1 |

Das als Ausgangsprodukt eingesetzte 6-Brom-9-(2-bromäthyl)-1,2,3,4-tetrahydro-carbazol-1-on erhält man aus 6-Brom-1,2,3,4-tetrahydro-carbazol-1-on (Fp.: 227°C) nach den Angaben des Beispiels 1 bezüglich Herstellung der Ausgangsprodukte mit einem Fp.: 126°C in einer Ausbeute von 88% d.Th.; Molgewicht: 371.

$C_{14}H_{13}Br_2NO$

| | C | H | Br | N | O |
|---|---|---|---|---|---|
| berechnet: | 45,2 | 3,5 | 43,1 | 3,8 | 4,3 |
| gefunden: | 45,0 | 3,4 | 42,8 | 3,6 | 4,5 |

Beispiel 5

Man setzt 6-Fluor-9-(2-bromäthyl)-1,2,3,4-tetrahydrocarbazol-1-on nach den Angaben des Beispiels 1 um und erhält 8-Fluor-2,4,5,6-tetrahydro-1H-pyrazino[3,2,1-jk]carbazol-Hydrochlorid.

Fp.: 303 bis 305°C; Ausbeute: 81% d.Th.; Molgewicht 264,5.

$C_{14}H_{13}FN_2.HCl$

| | Cl | H | F | N | $Cl^{\ominus}$ |
|---|---|---|---|---|---|
| berechnet: | 63,5 | 5,3 | 7,2 | 10,6 | 13,4 |
| gefunden: | 62,8 | 5,5 | 6,8 | 10,3 | 13,9 |

Das aus Ausgangsprodukt verwendete 6-Fluor-9-(2-bromäthyl)-1,2,3,4-tetrahydrocarbazol-1-on erhält man aus 6-Fluor-1,2,3,4-tetrahydrocarbazol-1-on (Fp.: 210°C) nach den Angaben des Beispiels 1 bezüglich Herstellung der Ausgangsprodukte mit einem Fp.: 131°C in einer Ausbeute von 76% d.Th.; Molgewicht: 310.

$C_{14}H_{13}BrFNO$

| | C | H | Br | F | N |
|---|---|---|---|---|---|
| berechnet: | 54,2 | 4,2 | 25,8 | 6,1 | 4,5 |
| gefunden: | 54,1 | 4,1 | 25,8 | 5,9 | 4,9 |

Beispiel 6

122 g (0,38 Mol) 6-Methyl-9-(β-methylsulfonyloxy-äthyl)-1,2,3,4-tetrahydrocarbazol-1-on werden in 650 ml (26 Mol) flüssigem Ammoniak bei 50°C 20 Stunden in einem Autoklaven erhitzt. Der nach dem Abdampfen des Ammoniaks bleibende Rückstand wird in einem Gemisch von 1000 ml Essigeseter und 500 ml Wasser suspendiert und so viel verdünnte Natronlauge zugesetzt, bis die wäßrige Schicht deutlich alkalisch reagiert. Man schüttelt kräftig durch, wäscht die organische Schicht mit Wasser, trocknet mit Natriumsulfat und fällt das Hydrochlorid des 8-Methyl-2,4,5,6-tetrahydro-1H-pyrazino[3,2,1-jk]carbazol durch Einleiten von trockenem Chlorwasserstoffgas aus. Man saugt ab und kristallisiert aus einem Gemisch Essigester-Äthanol (1 : 1) um.

Fp: 274—276°C, Ausbeute: 79,2 g (80% d.Th.)

$C_{15}H_{16}N_2$-HCl Molgewicht: 260,5.

Herstellung der Ausgangsprodukte:

6-Methyl-9-(2-hydroxyäthyl)-1,2,3,4-tetrahydrocarbazol-1-on: 199 g (1 Mol) 6-Methyl-1,2,3,4-tetrahydrocarbazol-1-on werden in 2000 ml Dimethylformamid gelöst vorgelegt und 5,6 g (0,1 Mol) Ätzkali, gelöst in 200 ml Wasser zugegeben. Dann werden 66,1 g ≡ 74,2 ml (1,5 Mol) Äthylenoxid bei Raumtemperatur zugegeben und innerhalb 30 Minuten auf 50°C hochgeheizt. Es entsteht ein Druck von max. 0,2—0,5 bar. Es wird 20 Stunden bei 50°C gerührt, das Lösungsmittel im Vakuum abdestilliert, der Rückstand mit einem Gemisch von 800 ml Wasser und 10 ml Eisessing verrührt, das kristallin gewordene Produkt abgesaugt, mit 500 ml Wasser gewaschen und im Vakuum bei 40°C getrocknet.

Ausbeute: 239,5 g (98,6% d.Th.); Fp.: 113—116°C.

$C_{15}H_{17}NO_2$ Molgewicht: 243.

6-Methyl-9-(β-methylsulfonyloxy-äthyl-)-1,2,3,4-tetrahydrocarbazol-1-on:

239,5 g (0,98 Mol) 6-Methyl-9-(2-hydroxyäthyl)-1,2,3,4-tetrahydrocarbazol-1-on werden in 2000 ml Toluol gelöst, 109,6 g (1,08 Mol) Triäthylamin zugegeben und 124,3 g (1,08 Mol) Methansulfochlorid innerhalb 15 Minuten zugetropft. Die Temperatur wird durch Kühlen bei 25—30°C gehalten. Anschließend wird 30 Minuten bei fallender Temperatur nachgerührt. Nach der Zugabe von 3000 ml Toluol, 500 ml Wasser und 15 ml Konz. Salzsäure wird auf 55°C erwärmt, die Toluolphase abgetrennt, mit 500 ml Wasser gewaschen und eingedampft. Der Rückstand wird aus 5 Liter Isopropanol umkristallisiert.

Ausbeute: 280 g (89% d.Th.); Fp.: 131—133°C Zers.

$C_6H_{19}NO_4S$ Molgewicht: 321

**Patentansprüche**

1. In 8-Stellung substituierte 2,4,5,6-Tetrahydro-1H-pyrazino[3,2,1-jk]carbazole der Formel I

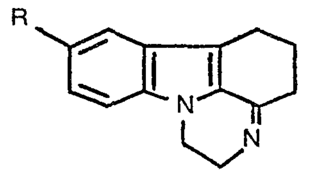

(I)

wobei R für eine Alkyl- oder Alkoxygruppe mit 1—4 Kohlenstoffatomen oder ein Fluor-, Chlor- oder Bromatom steht, und ihre pharmazeutisch annehmbaren Säureadditionssalze.

2. Verbindungen nach Anspruch 1 und ihre pharmazeutisch annehmbaren Säureadditionssalze, wobei R für eine Methyl- oder Methoxygruppe steht.

3. Verfahren zur Herstellung der Verbindungen nach den Ansprüchen 1 bis 2, dadurch gekennzeichnet, daß ein 6,9-disubstituiertes 1,2,3,4-Tetrahydrocarbazol-1-on der Formel II

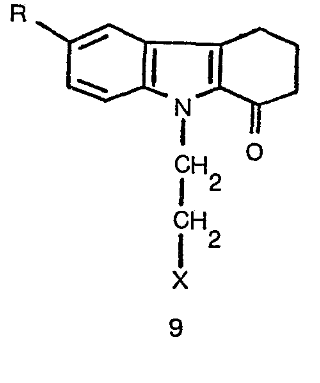

(II)

wobei R die in den Ansprüchen 1 bis 2 angegebene Bedeutung hat und X ein Brom-, Chlor- oder Jodatom oder den Rest der Formel —O—$SO_2$—R′ und R′ einen Alkylrest mit 1—3 Kohlenstoffatomen, Phenyl oder Tolyl bedeutet, mit Ammoniak unter Ringschluß umgesetzt wird.

4. Verfahren nach Anspruch 3, dadurch gekennzeichnet, daß die Umsetzung mit flüssigem Ammoniak durchgeführt wird.

5. Verfahren nach den Ansprüchen 3 oder 4, dadurch gekennzeichnet, daß die Umsetzung bei Temperaturen von 50 bis 100°C unter Rühren durchgeführt wird.

6. Psychotropes Mittel, dadurch gekennzeichnet, daß es als Wirkstoff eine Verbindung nach den Ansprüchen 1 oder 2 enthält.

7. 8-Methyl-2,4,5,6-tetrahydro-1H-pyrazino[3,2,1-jk]-carbazol und seine pharmazeutisch annehmbaren Säureadditionssalze.

8. 8-Methoxy-2,4,5,6-tetrahydro-1H-pyrazino[3,2-1-jk]-carbazol und seine pharmazeutisch annehmbaren Säureadditionssalze.

## Claims

1. 2,4,5,6-Tetrahydro-1H-pyrazino[3,2,1-jk]-carbazoles substituted in the 8-position, of the formula I

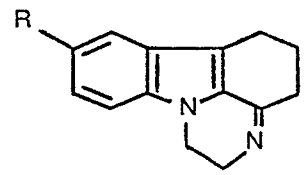

(I)

wherein R denotes an alkyl or alkoxy group having 1—4 carbon atoms or a fluorine, chlorine or bromine atom, and their pharmaceutically acceptable acid addition salts.

2. Compounds of Claim 1 and their pharmaceutically acceptable acid addition salts, wherein R denotes a methyl or methoxy group.

3. Process for preparing the compounds of Claims 1 to 2, characterised in that a 6,9-disubstituted 1,2,3,4-tetrahydrocarbazol-1-one of the formula II

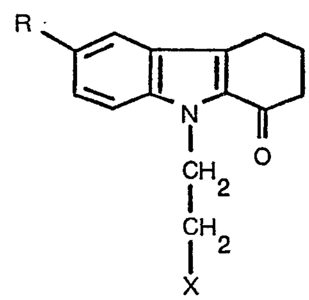

(II)

wherein R has the meaning indicated in Claims 1 to 2 and X denotes a bromine, chlorine or iodine atom or the radical of the formula —O—$SO_2$—R′ and R′ denotes an alkyl radical having 1—3 carbon atoms, phenyl or tolyl, is reacted with ammonia with ring closure.

4. The process of Claim 3, characterised in that the reaction is carried out with liquid ammonia.

5. The process of Claim 3 or 4, characterised in that the reaction is carried out at temperatures of from 50 to 100°C with stirring.

6. Psychotropic product, characterised in that it contains as the active substance a compound of Claim 1 or 2.

7. 8-Methyl-2,4,5,6-tetrahydro-1H-pyrazino[3,2,1-jk]carbazole and its pharmaceutically acceptable acid addition salts.

8. 8-Methoxy-2,4,5,6-tetrahydro-1H-pyrazino[3,2,1-jk]carbazole and its pharmaceutically acceptable acid addition salts.

**Revendications**

1. 2,4,5,6-Tétrahydro-1H-pyrazino[3,2,1-jk]carbazoles substitués à la position 8, de formule générale I

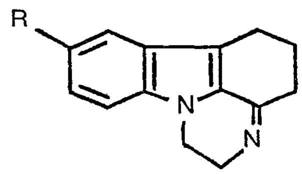

(I)

dans laquelle le symbole R représente un groupe alkyle ou alcoxy en $C_1$ à $C_4$ ou un atome de fluor, de chlore ou de brome, ainsi que leurs sels d'addition d'acides pharmaceutiquement acceptables.

2. Composés selon la revendication 1 et leurs sels d'addition d'acides pharmaceutiquement acceptables, dans lesquels R est un groupe méthyle ou méthoxy.

3. Procédé de préparation des composés selon la revendication 1 ou 2, caractérisé en ce que l'on fait réagir avec de l'ammoniac, pour réaliser la cyclisation, une 1,2,3,4-tétrahydrocarbazole-1-one disubstituée aux positions 6 et 9, de formule II

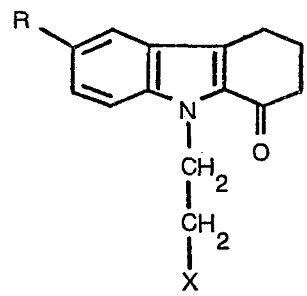

(II)

R ayant la signification donnée à la revendication 1 ou 2 et X désignant un atome de brome, de chlore ou d'iode ou un radical $—O—SO_2—R'$, R' étant un groupe alkyle en $C_1$ à $C_3$, phényle ou tolyle.

4. Procédé selon la revendication 3, caractérisé en ce que la réaction est effectuée avec de l'ammoniac liquide.

5. Procédé selon la revendication 3 ou 4, caractérisé en ce que la réaction est effectuée à des températures de 50 à 100°C avec agitation.

6. Produit psychotrope caractérisé en ce qu'il contient comme matière active un composé selon la revendication 1 ou 2.

7. Le 8-méthyl-2,4,5,6-tétrahydro-1H-pyrazino[3,2,1-jk]-carbazole et ses sels d'addition d'acides pharmaceutiquement acceptables.

8. Le 8-méthoxy-2,4,5,6-tétrahydro-1H-pyrazino[3,2,1-jk]-carbazole et ses sels d'addition d'acides pharmaceutiquement acceptables.